# EUROPEAN PATENT SPECIFICATION

(11) **EP 3 734 283 B1**
(45) Date of publication and mention of the grant of the patent: **14.06.2023**
(21) Application number: 18899192.1
(22) Date of filing: 10.07.2018
(51) Int. Cl.: G01N 33/68, G01N 33/48, G01N 33/53, G01N 21/76, G01N 33/564

(54) **APPLICATIONS OF BIOMARKER IN TERMS OF DETERMINING ANTIPHOSPHOLIPID SYNDROME AND REAGENT KIT OF BIOMARKER**
ANWENDUNGEN VON BIOMARKERN ZUR BESTIMMUNG DES ANTIPHOSPHOLIPID-SYNDROMS UND REAGENZIENKIT FÜR BIOMARKER
APPLICATIONS D'UN BIOMARQUEUR PAR RAPPORT À LA DÉTERMINATION DU SYNDROME DES ANTIPHOSPHOLIPIDES ET KIT DE RÉACTIFS DE BIOMARQUEUR

(30) Priority: 12.01.2018 CN 201810030852
(43) Date of publication of application: 04.11.2020
(73) Proprietor: GUANGZHOU KANGRUN BIOTECHNOLOGY CO., LTD, Guangzhou, Guangdong 511442 (CN)
(72) Inventor: YANG, Chengde, Guangzhou, Guangdong 511442 (CN); SHI, Hui, Guangzhou, Guangdong 511442 (CN); HUANG, Wenxi, Guangzhou, Guangdong 511442 (CN)
(74) Representative: Monteiro Alves, Inês
(86) International application number: PCT/CN2018/095067
(87) International publication number: WO 2019/136948

(56) References cited:
- WO-A1-2010/121714
- WO-A1-2011/022780
- WO-A1-2013/006156
- WO-A1-2017/122203
- WO-A1-2017/194779
- WO-A2-2005/054853
- CN-A- 108 226 534
- CN-U- 206 489 172
- US-A- 5 998 223
- US-A1- 2010 261 194
- MALIN ?STENSSON ET AL: "A Possible Mechanism behind Autoimmune Disorders Discovered By Genome-Wide Linkage and Association Analysis in Celiac Disease", PLOS ONE, vol. 8, no. 8, 2 August 2013 (2013-08-02), page e70174, XP055343972, DOI: 10.1371/journal.pone.0070174
- HAN Haibo et al.: "Preparation and identification of mouse anti-human myosin va polyclonal antibody", Chinese Journal of Cellular and Molecular Immunology, vol. 25, no. 5, 18 May 2005 (2005-05-18), pages 451-453, XP009522459, ISSN: 1007-8738, DOI: 10.13423/j.cnki.cjcmi.005016
- Venkaiah Betapudi , George Lominadze , Linda Hsi , Belinda Willard , Meifang Wu , Keith R McCrae: "Anti-beta 2GPI antibodies stimulate endothelial cell mi-croparticle release via a non-muscle myosin II motor protein-dependent pathway", Blood, vol. 122, no. 23, 28 August 2013 (2013-08-28), pages 3808-3817, XP055625069, DOI: 10.1182/blood-2013-03-490318
- YAMAZAKI REIJI ET AL: "Expression of Unconventional Myosin VI in Oligodendrocytes", NEUROCHEMICAL RESEARCH, PLENUM PRESS, NEW YORK, US, vol. 42, no. 12, 21 August 2017 (2017-08-21), pages 3372-3381, XP036365055, ISSN: 0364-3190, DOI: 10.1007/S11064-017-2377-7 [retrieved on 2017-08-21]

## Description

### TECHNICAL FIELD

The present disclosure relates to the technical field of immunization, and in particular to the use of antibodies to MYOSA protein as a biomarker in terms of determining antiphospholipid syndrome and use of reagent kit comprising the biomarker.

### BACKGROUND

APS, also known as antiphospholipid syndrome, is a series of syndromes clinically manifested by recurrent arteriovenous thrombosis or habitual abortion and accompanied by antiphospholipid antibody (APA) positive persistently, which were first described by Harris EN in 1985. The basic pathological change is intravascular thrombosis, arteriovenous vascular thrombosis at all levels can cause corresponding symptoms, and small placental vascular thrombosis can cause abortion. Therefore, thrombosis is the most prominent clinical manifestation of APS and the main reason for acquired thrombosis currently recognized.

Symptoms of APS, in the skin, include livedo reticularis, skin ulcers, thrombophlebitis, acral necrosis, livedo reticularis cerebral thrombosis (Sneddon syndrome), Behcet's disease and malignant atrophic papulosis rash. In the nervous system, they can include migraine, chorea, local cerebral ischemia, lupus sclerosis, Jamaican myelopathy, and Guillain-Barré syndrome. Symptoms of visceral infarction and thrombosis can include cerebral infarction, cerebral venous thrombosis, myocardial infarction, renal infarction, pulmonary infarction, liver Budd-Chiari syndrome, aseptic bone necrosis, habitual abortion, retinal arteriovenous thrombosis and arterial occlusion of extremities.

Document "Expression of Unconventional Myosin VI in Oligodendrocytes" analyzes the expression of Myo6 in oligodendrocytes. Yamazaki Reiji et al. performed immunofluorescence analysis on brains of adult normal and cuprizone-induced demyelination mice. Myo6 expression was detected in mature oligodendrocytes and oligodendrocyte progenitor cells in the cerebellum and corpus callosum. To compare temporal expression patterns of myosin superfamily members in vitro, double immunostainings using anti-Myo6, myosin Va (Myo5a), or Myo1d with each stage-specific oligodendrocyte marker antibody were performed. In cultured oligodendrocytes, although Myo1d was found only in mature oligodendrocytes, Myo6 and Myo5a signals were detected in all stages.

Document WO2017122203A1 discloses methods of determining prognosis of a subject diagnosed with sepsis, by determining the baseline levels of T-cell senescence in the subject. Also provided are methods of treating a subject diagnosed with sepsis, by monitoring the subject diagnosed with sepsis and treating the subject which exhibits high baseline levels of T-cell senescence.

WO2013/006156 discloses autoimmune disease detection using improved anti-nuclear antibody assays.

WO2017/194779 relates to the use of anti-CD26 antibodies as markers for the screening, diagnosis or monitoring of human subjects having, or suspected to have, an autoimmune or inflammatory disease.

It is necessary to provide biomarkers for determining the antiphospholipid syndrome for the judgment of APS.

### SUMMARY

The purpose of the present invention is the use of a biomarker for determining the antiphospholipid syndrome and a reagent kit comprising such biomarker.

The above object of the present disclosure is achieved by the following technical means.

In particular, the present invention relates to the use of antibodies to MYOSA protein as a biomarker in the *in vitro* determination of antiphospholipid syndrome in biological samples selected from serum, plasma or tissue fluid obtained from a subject.

Preferably, the antibodies to the MYOSA protein include at least one of IgG, IgM, or IgA.

Preferably, the antiphospholipid syndrome is primary antiphospholipid syndrome.

Another preferred, the antiphospholipid syndrome is secondary antiphospholipid syndrome.

Another preferred, the primary antiphospholipid syndrome and secondary antiphospholipid syndrome are determined simultaneously.

Preferably, the sequence of MYOSA protein is,

Met Ala Ala Ser Glu Leu Tyr Thr Lys Phe Ala Arg Val Trp Ile Pro

1               5                   10                  15
Asp Pro Glu Glu Val Trp Lys Ser Ala Glu Leu Leu Lys Asp Tyr Lys
            20                  25                  30
Pro Gly Asp Lys Val Leu Leu Leu His Leu Glu Glu Gly Lys Asp Leu
        35                  40                  45
Glu Tyr His Leu Asp Pro Lys Thr Lys Glu Leu Pro His Leu Arg Asn
    50                  55                  60
Pro Asp Ile Leu Val Gly Glu Asn Asp Leu Thr Ala Leu Ser Tyr Leu
65                  70                  75                  80
His Glu Pro Ala Val Leu His Asn Leu Arg Val Arg Phe Ile Asp Ser
                85                  90                  95
Lys Leu Ile Tyr Thr Tyr Cys Gly Ile Val Leu Val Ala Ile Asn Pro
            100                 105                 110
Tyr Glu Gln Leu Pro Ile Tyr Gly Glu Asp Ile Ile Asn Ala Tyr Ser
        115                 120                 125
Gly Gln Asn Met Gly Asp Met Asp Pro His Ile Phe Ala Val Ala Glu
    130                 135                 140
Glu Ala Tyr Lys Gln Met Ala Arg Asp Glu Arg Asn Gln Ser Ile Ile
145                 150                 155                 160
Val Ser Gly Glu Ser Gly Ala Gly Lys Thr Val Ser Ala Lys Tyr Ala
                165                 170                 175
Met Arg Tyr Phe Ala Thr Val Ser Gly Ser Ala Ser Glu Ala Asn Val
            180                 185                 190
Glu Glu Lys Val Leu Ala Ser Asn Pro Ile Met Glu Ser Ile Gly Asn
        195                 200                 205
Ala Lys Thr Thr Arg Asn Asp Asn Ser Ser Arg Phe Gly Lys Tyr Ile
    210                 215                 220
Glu Ile Gly Phe Asp Lys Arg Tyr Arg Ile Ile Gly Ala Asn Met Arg
225                 230                 235                 240

Thr Tyr Leu Leu Glu Lys Ser Arg Val Val Phe Gln Ala Glu Glu Glu
                245                 250                 255
Arg Asn Tyr His Ile Phe Tyr Gln Leu Cys Ala Ser Ala Lys Leu Pro
            260                 265                 270
Glu Phe Lys Met Leu Arg Leu Gly Asn Ala Asp Asn Phe Asn Tyr Thr
        275                 280                 285
Lys Gln Gly Gly Ser Pro Val Ile Glu Gly Val Asp Asp Ala Lys Glu
    290                 295                 300
Met Ala His Thr Arg Gln Ala Cys Thr Leu Leu Gly Ile Ser Glu Ser
305                 310                 315                 320
His Gln Met Gly Ile Phe Arg Ile Leu Ala Gly Ile Leu His Leu Gly
                325                 330                 335
Asn Val Gly Phe Thr Ser Arg Asp Ala Asp Ser Cys Thr Ile Pro Pro
            340                 345                 350
Lys His Glu Pro Leu Cys Ile Phe Cys Glu Leu Met Gly Val Asp Tyr
        355                 360                 365
Glu Glu Met Cys His Trp Leu Cys His Arg Lys Leu Ala Thr Ala Thr
    370                 375                 380
Glu Thr Tyr Ile Lys Pro Ile Ser Lys Leu Gln Ala Thr Asn Ala Arg
385                 390                 395                 400
Asp Ala Leu Ala Lys His Ile Tyr Ala Lys Leu Phe Asn Trp Ile Val
                405                 410                 415
Asp Asn Val Asn Gln Ala Leu His Ser Ala Val Lys Gln His Ser Phe
            420                 425                 430
Ile Gly Val Leu Asp Ile Tyr Gly Phe Glu Thr Phe Glu Ile Asn Ser
        435                 440                 445
Phe Glu Gln Phe Cys Ile Asn Tyr Ala Asn Glu Lys Leu Gln Gln Gln
    450                 455                 460
Phe Asn Met His Val Phe Lys Leu Glu Gln Glu Glu Tyr Met Lys Glu

465                 470                 475                 480
Gln Ile Pro Trp Thr Leu Ile Asp Phe Tyr Asp Asn Gln Pro Cys Ile
                485                 490                 495
Asn Leu Ile Glu Ser Lys Leu Gly Ile Leu Asp Leu Leu Asp Glu Glu
            500                 505                 510
Cys Lys Met Pro Lys Gly Thr Asp Asp Thr Trp Ala Gln Lys Leu Tyr
        515                 520                 525
Asn Thr His Leu Asn Lys Cys Ala Leu Phe Glu Lys Pro Arg Leu Ser
    530                 535                 540
Asn Lys Ala Phe Ile Ile Gln His Phe Ala Asp Lys Val Glu Tyr Gln
545                 550                 555                 560
Cys Glu Gly Phe Leu Glu Lys Asn Lys Asp Thr Val Phe Glu Glu Gln
                565                 570                 575
Ile Lys Val Leu Lys Ser Ser Lys Phe Lys Met Leu Pro Glu Leu Phe
            580                 585                 590
Gln Asp Asp Glu Lys Ala Ile Ser Pro Thr Ser Ala Thr Ser Ser Gly
        595                 600                 605
Arg Thr Pro Leu Thr Arg Thr Pro Ala Lys Pro Thr Lys Gly Arg Pro
    610                 615                 620
Gly Gln Met Ala Lys Glu His Lys Lys Thr Val Gly His Gln Phe Arg
625                 630                 635                 640
Asn Ser Leu His Leu Leu Met Glu Thr Leu Asn Ala Thr Thr Pro His
                645                 650                 655
Tyr Val Arg Cys Ile Lys Pro Asn Asp Phe Lys Phe Pro Phe Thr Phe
            660                 665                 670
Asp Glu Lys Arg Ala Val Gln Gln Leu Arg Ala Cys Gly Val Leu Glu
        675                 680                 685
Thr Ile Arg Ile Ser Ala Ala Gly Phe Pro Ser Arg Trp Thr Tyr Gln
    690                 695                 700

Glu Phe Phe Ser Arg Tyr Arg Val Leu Met Lys Gln Lys Asp Val Leu
705                 710                 715                 720
Ser Asp Arg Lys Gln Thr Cys Lys Asn Val Leu Glu Lys Leu Ile Leu
                725                 730                 735
Asp Lys Asp Lys Tyr Gln Phe Gly Lys Thr Lys Ile Phe Phe Arg Ala
            740                 745                 750
Gly Gln Val Ala Tyr Leu Glu Lys Leu Arg Ala Asp Lys Leu Arg Ala
        755                 760                 765
Ala Cys Ile Arg Ile Gln Lys Thr Ile Arg Gly Trp Leu Leu Arg Lys
    770                 775                 780
Lys Tyr Leu Arg Met Arg Lys Ala Ala Ile Thr Met Gln Arg Tyr Val
785                 790                 795                 800
Arg Gly Tyr Gln Ala Arg Cys Tyr Ala Lys Phe Leu Arg Arg Thr Lys
                805                 810                 815
Ala Ala Thr Ile Ile Gln Lys Tyr Trp Arg Met Tyr Val Val Arg Arg
            820                 825                 830
Arg Tyr Lys Ile Arg Arg Ala Ala Thr Ile Val Leu Gln Ser Tyr Leu
        835                 840                 845
Arg Gly Phe Leu Ala Arg Asn Arg Tyr Arg Lys Ile Leu Arg Glu His
    850                 855                 860
Lys Ala Val Ile Ile Gln Lys Arg Val Arg Gly Trp Leu Ala Arg Thr
865                 870                 875                 880
His Tyr Lys Arg Ser Met His Ala Ile Ile Tyr Leu Gln Cys Cys Phe
                885                 890                 895
Arg Arg Met Met Ala Lys Arg Glu Leu Lys Lys Leu Lys Ile Glu Ala
            900                 905                 910
Arg Ser Val Glu Arg Tyr Lys Lys Leu His Ile Gly Met Glu Asn Lys
        915                 920                 925
Ile Met Gln Leu Gln Arg Lys Val Asp Glu Gln Asn Lys Asp Tyr Lys

    930                 935                 940
Cys Leu Val Glu Lys Leu Thr Asn Leu Glu Gly Ile Tyr Asn Ser Glu
945                 950                 955                 960
Thr Glu Lys Leu Arg Ser Asp Leu Glu Arg Leu Gln Leu Ser Glu Glu
                965                 970                 975
Glu Ala Lys Val Ala Thr Gly Arg Val Leu Ser Leu Gln Glu Glu Ile
            980                 985                 990
Ala Lys Leu Arg Lys Asp Leu Glu Gln Thr Arg Ser Glu Lys Lys Cys
        995                 1000                1005
Ile Glu Glu His Ala Asp Arg Tyr Lys Gln Glu Thr Glu Gln Leu
    1010                1015                1020
Val Ser Asn Leu Lys Glu Glu Asn Thr Leu Leu Lys Gln Glu Lys
    1025                1030                1035
Glu Ala Leu Asn His Arg Ile Val Gln Gln Ala Lys Glu Met Thr
    1040                1045                1050
Glu Thr Met Glu Lys Lys Leu Val Glu Glu Thr Lys Gln Leu Glu
    1055                1060                1065
Leu Asp Leu Asn Asp Glu Arg Leu Arg Tyr Gln Asn Leu Leu Asn
    1070                1075                1080
Glu Phe Ser Arg Leu Glu Glu Arg Tyr Asp Asp Leu Lys Glu Glu
    1085                1090                1095
Met Thr Leu Met Val His Val Pro Lys Pro Gly His Lys Arg Thr
    1100                1105                1110
Asp Ser Thr His Ser Ser Asn Glu Ser Glu Tyr Ile Phe Ser Ser
    1115                1120                1125
Glu Ile Ala Glu Met Glu Asp Ile Pro Ser Arg Thr Glu Glu Pro
    1130                1135                1140
Ser Glu Lys Lys Val Pro Leu Asp Met Ser Leu Phe Leu Lys Leu
    1145                1150                1155

Gln Lys Arg Val Thr Glu Leu Glu Gln Glu Lys Gln Val Met Gln
    1160                1165                1170
Asp Glu Leu Asp Arg Lys Glu Glu Gln Val Leu Arg Ser Lys Ala
    1175                1180                1185
Lys Glu Glu Glu Arg Pro Gln Ile Arg Gly Ala Glu Leu Glu Tyr
    1190                1195                1200
Glu Ser Leu Lys Arg Gln Glu Leu Glu Ser Glu Asn Lys Lys Leu
    1205                1210                1215
Lys Asn Glu Leu Asn Glu Leu Arg Lys Ala Leu Ser Glu Lys Ser
    1220                1225                1230
Ala Pro Glu Val Thr Ala Pro Gly Ala Pro Ala Tyr Arg Val Leu
    1235                1240                1245
Met Glu Gln Leu Thr Ser Val Ser Glu Glu Leu Asp Val Arg Lys
    1250                1255                1260
Glu Glu Val Leu Ile Leu Arg Ser Gln Leu Val Ser Gln Lys Glu
    1265                1270                1275
Ala Ile Gln Pro Lys Asp Asp Lys Asn Thr Met Thr Asp Ser Thr
    1280                1285                1290
Ile Leu Leu Glu Asp Val Gln Lys Met Lys Asp Lys Gly Glu Ile
    1295                1300                1305
Ala Gln Ala Tyr Ile Gly Leu Lys Glu Thr Asn Arg Ser Ser Ala
    1310                1315                1320
Leu Asp Tyr His Glu Leu Asn Glu Asp Gly Glu Leu Trp Leu Val
    1325                1330                1335
Tyr Glu Gly Leu Lys Gln Ala Asn Arg Leu Leu Glu Ser Gln Leu
    1340                1345                1350
Gln Ser Gln Lys Arg Ser His Glu Asn Glu Ala Glu Ala Leu Arg
    1355                1360                1365
Gly Glu Ile Gln Ser Leu Lys Glu Glu Asn Asn Arg Gln Gln Gln

1370                1375                1380

Leu Leu Ala Gln Asn Leu Gln Leu Pro Pro Glu Ala Arg Ile Glu

1385                1390                1395

Ala Ser Leu Gln His Glu Ile Thr Arg Leu Thr Asn Glu Asn Leu

1400                1405                1410

Asp Leu Met Glu Gln Leu Glu Lys Gln Asp Lys Thr Val Arg Lys

1415                1420                1425

Leu Lys Lys Gln Leu Lys Val Phe Ala Lys Lys Ile Gly Glu Leu

1430                1435                1440

Glu Val Gly Gln Met Glu Asn Ile Ser Pro Gly Gln Ile Ile Asp

1445                1450                1455

Glu Pro Ile Arg Pro Val Asn Ile Pro Arg Lys Glu Lys Asp Phe

1460                1465                1470

Gln Gly Met Leu Glu Tyr Lys Lys Glu Asp Glu Gln Lys Leu Val

1475                1480                1485

Lys Asn Leu Ile Leu Glu Leu Lys Pro Arg Gly Val Ala Val Asn

1490                1495                1500

Leu Ile Pro Gly Leu Pro Ala Tyr Ile Leu Phe Met Cys Val Arg

1505                1510                1515

His Ala Asp Tyr Leu Asn Asp Asp Gln Lys Val Arg Ser Leu Leu

1520                1525                1530

Thr Ser Thr Ile Asn Ser Ile Lys Lys Val Leu Lys Lys Arg Gly

1535                1540                1545

Asp Asp Phe Glu Thr Val Ser Phe Trp Leu Ser Asn Thr Cys Arg

1550                1555                1560

Phe Leu His Cys Leu Lys Gln Tyr Ser Gly Glu Glu Gly Phe Met

1565                1570                1575

Lys His Asn Thr Ser Arg Gln Asn Glu His Cys Leu Thr Asn Phe

1580                1585                1590

Asp Leu Ala Glu Tyr Arg Gln Val Leu Ser Asp Leu Ala Ile Gln
    1595                1600                1605
Ile Tyr Gln Gln Leu Val Arg Val Leu Glu Asn Ile Leu Gln Pro
    1610                1615                1620
Met Ile Val Ser Gly Met Leu Glu His Glu Thr Ile Gln Gly Val
    1625                1630                1635
Ser Gly Val Lys Pro Thr Gly Leu Arg Lys Arg Thr Ser Ser Ile
    1640                1645                1650
Ala Asp Glu Gly Thr Tyr Thr Leu Asp Ser Ile Leu Arg Gln Leu
    1655                1660                1665
Asn Ser Phe His Ser Val Met Cys Gln His Gly Met Asp Pro Glu
    1670                1675                1680
Leu Ile Lys Gln Val Val Lys Gln Met Phe Tyr Ile Ile Gly Ala
    1685                1690                1695
Ile Thr Leu Asn Asn Leu Leu Leu Arg Lys Asp Met Cys Ser Trp
    1700                1705                1710
Ser Lys Gly Met Gln Ile Arg Tyr Asn Val Ser Gln Leu Glu Glu
    1715                1720                1725
Trp Leu Arg Asp Lys Asn Leu Met Asn Ser Gly Ala Lys Glu Thr
    1730                1735                1740
Leu Glu Pro Leu Ile Gln Ala Ala Gln Leu Leu Gln Val Lys Lys
    1745                1750                1755
Lys Thr Asp Asp Asp Ala Glu Ala Ile Cys Ser Met Cys Asn Ala
    1760                1765                1770
Leu Thr Thr Ala Gln Ile Val Lys Val Leu Asn Leu Tyr Thr Pro
    1775                1780                1785
Val Asn Glu Phe Glu Glu Arg Val Ser Val Ser Phe Ile Arg Thr
    1790                1795                1800
Ile Gln Met Arg Leu Arg Asp Arg Lys Asp Ser Pro Gln Leu Leu

1805                    1810                    1815
Met Asp  Ala Lys His Ile Phe  Pro Val Thr Phe Pro  Phe Asn Pro
    1820                    1825                    1830
Ser Ser  Leu Ala Leu Glu Thr  Ile Gln Ile Pro Ala  Ser Leu Gly
    1835                    1840                    1845
Leu Gly  Phe Ile Ser Arg Val
1850                    1855.

The present invention also provides the use of a reagent kit in the in vitro determination of antiphopholipid syndrome, the reagent kit comprising MYOSA protein, wherein the presence of antibodies to MYOSA protein in biological samples are detected, the biological samples being selected from serum, plasma or tissue fluid obtained from a subj ect.

Further, in the above use of reagent kit in the in vitro determination of antiphopholipid syndrome, the antibodies to MYOSA protein include at least one of IgG, IgM or IgA.

Further, in the above use of reagent kit in the in vitro determination of antiphopholipid syndrome, the antibodies to MYOSA protein are detected by a detection method selected from any one of ELISA, chemiluminescence or POCT.

The present disclosure provides a new biomarker to identify antiphospholipid syndrome and a new application direction which is of great significance for the identification of antiphospholipid syndrome.

### BRIEF DESCRIPTION OF THE DRAWINGS

FIG. 1 is a graph showing the different results of antibodies to MYOSA protein in the APS group and the disease control group.
FIG. 2 is a schematic diagram of the results of the significant difference between the antibodies to MY05A protein in the APS group and in the healthy control group.
FIG. 3 is a schematic diagram of the difference between the antibodies to MY05A protein in the primary APS and in the secondary APS group.

### DESCRIPTION OF THE EMBODIMENTS

The present invention is further described in conjunction with the following embodiments.

### Embodiment 1

Use of antibodies to MYOSA protein as a biomarker in the in vitro determination of antiphospholipid syndrome, wherein antibodies to the MYOSA protein include at least one of IgG, IgM, or IgA.

Use of antibodies to MYOSA protein as a biomarker in the in vitro determination of antiphospholipid syndrome to identify primary antiphospholipid syndrome, secondary antiphospholipid syndrome or to identify primary antiphospholipid syndrome and secondary antiphospholipid syndrome simultaneously.

The sequence of MYOSA protein is,

Met Ala Ala Ser Glu Leu Tyr Thr Lys Phe Ala Arg Val Trp Ile Pro
1               5                   10                  15
Asp Pro Glu Glu Val Trp Lys Ser Ala Glu Leu Leu Lys Asp Tyr Lys
            20                  25                  30
Pro Gly Asp Lys Val Leu Leu Leu His Leu Glu Glu Gly Lys Asp Leu
        35                  40                  45
Glu Tyr His Leu Asp Pro Lys Thr Lys Glu Leu Pro His Leu Arg Asn
    50                  55                  60
Pro Asp Ile Leu Val Gly Glu Asn Asp Leu Thr Ala Leu Ser Tyr Leu
65                  70                  75                  80
His Glu Pro Ala Val Leu His Asn Leu Arg Val Arg Phe Ile Asp Ser
                85                  90                  95
Lys Leu Ile Tyr Thr Tyr Cys Gly Ile Val Leu Val Ala Ile Asn Pro
            100                 105                 110
Tyr Glu Gln Leu Pro Ile Tyr Gly Glu Asp Ile Ile Asn Ala Tyr Ser
        115                 120                 125
Gly Gln Asn Met Gly Asp Met Asp Pro His Ile Phe Ala Val Ala Glu
    130                 135                 140

Glu Ala Tyr Lys Gln Met Ala Arg Asp Glu Arg Asn Gln Ser Ile Ile
145                 150                 155                 160
Val Ser Gly Glu Ser Gly Ala Gly Lys Thr Val Ser Ala Lys Tyr Ala
                165                 170                 175
Met Arg Tyr Phe Ala Thr Val Ser Gly Ser Ala Ser Glu Ala Asn Val
            180                 185                 190
Glu Glu Lys Val Leu Ala Ser Asn Pro Ile Met Glu Ser Ile Gly Asn
        195                 200                 205
Ala Lys Thr Thr Arg Asn Asp Asn Ser Ser Arg Phe Gly Lys Tyr Ile
    210                 215                 220
Glu Ile Gly Phe Asp Lys Arg Tyr Arg Ile Ile Gly Ala Asn Met Arg
225                 230                 235                 240
Thr Tyr Leu Leu Glu Lys Ser Arg Val Val Phe Gln Ala Glu Glu Glu
                245                 250                 255
Arg Asn Tyr His Ile Phe Tyr Gln Leu Cys Ala Ser Ala Lys Leu Pro
            260                 265                 270
Glu Phe Lys Met Leu Arg Leu Gly Asn Ala Asp Asn Phe Asn Tyr Thr
        275                 280                 285
Lys Gln Gly Gly Ser Pro Val Ile Glu Gly Val Asp Asp Ala Lys Glu
    290                 295                 300
Met Ala His Thr Arg Gln Ala Cys Thr Leu Leu Gly Ile Ser Glu Ser
305                 310                 315                 320
His Gln Met Gly Ile Phe Arg Ile Leu Ala Gly Ile Leu His Leu Gly
                325                 330                 335
Asn Val Gly Phe Thr Ser Arg Asp Ala Asp Ser Cys Thr Ile Pro Pro
            340                 345                 350
Lys His Glu Pro Leu Cys Ile Phe Cys Glu Leu Met Gly Val Asp Tyr
        355                 360                 365
Glu Glu Met Cys His Trp Leu Cys His Arg Lys Leu Ala Thr Ala Thr

    370                 375                 380
Glu Thr Tyr Ile Lys Pro Ile Ser Lys Leu Gln Ala Thr Asn Ala Arg
385                 390                 395                 400
Asp Ala Leu Ala Lys His Ile Tyr Ala Lys Leu Phe Asn Trp Ile Val
                405                 410                 415
Asp Asn Val Asn Gln Ala Leu His Ser Ala Val Lys Gln His Ser Phe
            420                 425                 430
Ile Gly Val Leu Asp Ile Tyr Gly Phe Glu Thr Phe Glu Ile Asn Ser
        435                 440                 445
Phe Glu Gln Phe Cys Ile Asn Tyr Ala Asn Glu Lys Leu Gln Gln Gln
    450                 455                 460
Phe Asn Met His Val Phe Lys Leu Glu Gln Glu Glu Tyr Met Lys Glu
465                 470                 475                 480
Gln Ile Pro Trp Thr Leu Ile Asp Phe Tyr Asp Asn Gln Pro Cys Ile
                485                 490                 495
Asn Leu Ile Glu Ser Lys Leu Gly Ile Leu Asp Leu Leu Asp Glu Glu
            500                 505                 510
Cys Lys Met Pro Lys Gly Thr Asp Asp Thr Trp Ala Gln Lys Leu Tyr
        515                 520                 525
Asn Thr His Leu Asn Lys Cys Ala Leu Phe Glu Lys Pro Arg Leu Ser
    530                 535                 540
Asn Lys Ala Phe Ile Ile Gln His Phe Ala Asp Lys Val Glu Tyr Gln
545                 550                 555                 560
Cys Glu Gly Phe Leu Glu Lys Asn Lys Asp Thr Val Phe Glu Glu Gln
                565                 570                 575
Ile Lys Val Leu Lys Ser Ser Lys Phe Lys Met Leu Pro Glu Leu Phe
            580                 585                 590
Gln Asp Asp Glu Lys Ala Ile Ser Pro Thr Ser Ala Thr Ser Ser Gly
        595                 600                 605

Arg Thr Pro Leu Thr Arg Thr Pro Ala Lys Pro Thr Lys Gly Arg Pro
    610                 615                 620
Gly Gln Met Ala Lys Glu His Lys Lys Thr Val Gly His Gln Phe Arg
625                 630                 635                 640
Asn Ser Leu His Leu Leu Met Glu Thr Leu Asn Ala Thr Thr Pro His
                645                 650                 655
Tyr Val Arg Cys Ile Lys Pro Asn Asp Phe Lys Phe Pro Phe Thr Phe
            660                 665                 670
Asp Glu Lys Arg Ala Val Gln Gln Leu Arg Ala Cys Gly Val Leu Glu
        675                 680                 685
Thr Ile Arg Ile Ser Ala Ala Gly Phe Pro Ser Arg Trp Thr Tyr Gln
    690                 695                 700
Glu Phe Phe Ser Arg Tyr Arg Val Leu Met Lys Gln Lys Asp Val Leu
705                 710                 715                 720
Ser Asp Arg Lys Gln Thr Cys Lys Asn Val Leu Glu Lys Leu Ile Leu
                725                 730                 735
Asp Lys Asp Lys Tyr Gln Phe Gly Lys Thr Lys Ile Phe Phe Arg Ala
            740                 745                 750
Gly Gln Val Ala Tyr Leu Glu Lys Leu Arg Ala Asp Lys Leu Arg Ala
        755                 760                 765
Ala Cys Ile Arg Ile Gln Lys Thr Ile Arg Gly Trp Leu Leu Arg Lys
    770                 775                 780
Lys Tyr Leu Arg Met Arg Lys Ala Ala Ile Thr Met Gln Arg Tyr Val
785                 790                 795                 800
Arg Gly Tyr Gln Ala Arg Cys Tyr Ala Lys Phe Leu Arg Arg Thr Lys
                805                 810                 815
Ala Ala Thr Ile Ile Gln Lys Tyr Trp Arg Met Tyr Val Val Arg Arg
            820                 825                 830
Arg Tyr Lys Ile Arg Arg Ala Ala Thr Ile Val Leu Gln Ser Tyr Leu

        835                 840                 845
Arg Gly Phe Leu Ala Arg Asn Arg Tyr Arg Lys Ile Leu Arg Glu His
    850                 855                 860
Lys Ala Val Ile Ile Gln Lys Arg Val Arg Gly Trp Leu Ala Arg Thr
865                 870                 875                 880
His Tyr Lys Arg Ser Met His Ala Ile Ile Tyr Leu Gln Cys Cys Phe
                885                 890                 895
Arg Arg Met Met Ala Lys Arg Glu Leu Lys Lys Leu Lys Ile Glu Ala
            900                 905                 910
Arg Ser Val Glu Arg Tyr Lys Lys Leu His Ile Gly Met Glu Asn Lys
        915                 920                 925
Ile Met Gln Leu Gln Arg Lys Val Asp Glu Gln Asn Lys Asp Tyr Lys
    930                 935                 940
Cys Leu Val Glu Lys Leu Thr Asn Leu Glu Gly Ile Tyr Asn Ser Glu
945                 950                 955                 960
Thr Glu Lys Leu Arg Ser Asp Leu Glu Arg Leu Gln Leu Ser Glu Glu
                965                 970                 975
Glu Ala Lys Val Ala Thr Gly Arg Val Leu Ser Leu Gln Glu Glu Ile
            980                 985                 990
Ala Lys Leu Arg Lys Asp Leu Glu Gln Thr Arg Ser Glu Lys Lys Cys
        995                 1000                1005
Ile Glu Glu His Ala Asp Arg Tyr Lys Gln Glu Thr Glu Gln Leu
    1010                1015                1020
Val Ser Asn Leu Lys Glu Glu Asn Thr Leu Leu Lys Gln Glu Lys
    1025                1030                1035
Glu Ala Leu Asn His Arg Ile Val Gln Gln Ala Lys Glu Met Thr
    1040                1045                1050
Glu Thr Met Glu Lys Lys Leu Val Glu Glu Thr Lys Gln Leu Glu
    1055                1060                1065

Leu Asp Leu Asn Asp Glu Arg Leu Arg Tyr Gln Asn Leu Leu Asn
    1070                1075                1080
Glu Phe Ser Arg Leu Glu Glu Arg Tyr Asp Asp Leu Lys Glu Glu
    1085                1090                1095
Met Thr Leu Met Val His Val Pro Lys Pro Gly His Lys Arg Thr
    1100                1105                1110
Asp Ser Thr His Ser Ser Asn Glu Ser Glu Tyr Ile Phe Ser Ser
    1115                1120                1125
Glu Ile Ala Glu Met Glu Asp Ile Pro Ser Arg Thr Glu Glu Pro
    1130                1135                1140
Ser Glu Lys Lys Val Pro Leu Asp Met Ser Leu Phe Leu Lys Leu
    1145                1150                1155
Gln Lys Arg Val Thr Glu Leu Glu Gln Glu Lys Gln Val Met Gln
    1160                1165                1170
Asp Glu Leu Asp Arg Lys Glu Glu Gln Val Leu Arg Ser Lys Ala
    1175                1180                1185
Lys Glu Glu Glu Arg Pro Gln Ile Arg Gly Ala Glu Leu Glu Tyr
    1190                1195                1200
Glu Ser Leu Lys Arg Gln Glu Leu Glu Ser Glu Asn Lys Lys Leu
    1205                1210                1215
Lys Asn Glu Leu Asn Glu Leu Arg Lys Ala Leu Ser Glu Lys Ser
    1220                1225                1230
Ala Pro Glu Val Thr Ala Pro Gly Ala Pro Ala Tyr Arg Val Leu
    1235                1240                1245
Met Glu Gln Leu Thr Ser Val Ser Glu Glu Leu Asp Val Arg Lys
    1250                1255                1260
Glu Glu Val Leu Ile Leu Arg Ser Gln Leu Val Ser Gln Lys Glu
    1265                1270                1275
Ala Ile Gln Pro Lys Asp Asp Lys Asn Thr Met Thr Asp Ser Thr

1280                1285                1290
Ile Leu Leu Glu Asp Val Gln Lys Met Lys Asp Lys Gly Glu Ile
1295                1300                1305
Ala Gln Ala Tyr Ile Gly Leu Lys Glu Thr Asn Arg Ser Ser Ala
1310                1315                1320
Leu Asp Tyr His Glu Leu Asn Glu Asp Gly Glu Leu Trp Leu Val
1325                1330                1335
Tyr Glu Gly Leu Lys Gln Ala Asn Arg Leu Leu Glu Ser Gln Leu
1340                1345                1350
Gln Ser Gln Lys Arg Ser His Glu Asn Glu Ala Glu Ala Leu Arg
1355                1360                1365
Gly Glu Ile Gln Ser Leu Lys Glu Glu Asn Asn Arg Gln Gln Gln
1370                1375                1380
Leu Leu Ala Gln Asn Leu Gln Leu Pro Pro Glu Ala Arg Ile Glu
1385                1390                1395
Ala Ser Leu Gln His Glu Ile Thr Arg Leu Thr Asn Glu Asn Leu
1400                1405                1410
Asp Leu Met Glu Gln Leu Glu Lys Gln Asp Lys Thr Val Arg Lys
1415                1420                1425
Leu Lys Lys Gln Leu Lys Val Phe Ala Lys Lys Ile Gly Glu Leu
1430                1435                1440
Glu Val Gly Gln Met Glu Asn Ile Ser Pro Gly Gln Ile Ile Asp
1445                1450                1455
Glu Pro Ile Arg Pro Val Asn Ile Pro Arg Lys Glu Lys Asp Phe
1460                1465                1470
Gln Gly Met Leu Glu Tyr Lys Lys Glu Asp Glu Gln Lys Leu Val
1475                1480                1485
Lys Asn Leu Ile Leu Glu Leu Lys Pro Arg Gly Val Ala Val Asn
1490                1495                1500

Leu Ile Pro Gly Leu Pro Ala Tyr Ile Leu Phe Met Cys Val Arg
    1505                1510                1515
His Ala Asp Tyr Leu Asn Asp Asp Gln Lys Val Arg Ser Leu Leu
    1520                1525                1530
Thr Ser Thr Ile Asn Ser Ile Lys Lys Val Leu Lys Lys Arg Gly
    1535                1540                1545
Asp Asp Phe Glu Thr Val Ser Phe Trp Leu Ser Asn Thr Cys Arg
    1550                1555                1560
Phe Leu His Cys Leu Lys Gln Tyr Ser Gly Glu Glu Gly Phe Met
    1565                1570                1575
Lys His Asn Thr Ser Arg Gln Asn Glu His Cys Leu Thr Asn Phe
    1580                1585                1590
Asp Leu Ala Glu Tyr Arg Gln Val Leu Ser Asp Leu Ala Ile Gln
    1595                1600                1605
Ile Tyr Gln Gln Leu Val Arg Val Leu Glu Asn Ile Leu Gln Pro
    1610                1615                1620
Met Ile Val Ser Gly Met Leu Glu His Glu Thr Ile Gln Gly Val
    1625                1630                1635
Ser Gly Val Lys Pro Thr Gly Leu Arg Lys Arg Thr Ser Ser Ile
    1640                1645                1650
Ala Asp Glu Gly Thr Tyr Thr Leu Asp Ser Ile Leu Arg Gln Leu
    1655                1660                1665
Asn Ser Phe His Ser Val Met Cys Gln His Gly Met Asp Pro Glu
    1670                1675                1680
Leu Ile Lys Gln Val Val Lys Gln Met Phe Tyr Ile Ile Gly Ala
    1685                1690                1695
Ile Thr Leu Asn Asn Leu Leu Leu Arg Lys Asp Met Cys Ser Trp
    1700                1705                1710
Ser Lys Gly Met Gln Ile Arg Tyr Asn Val Ser Gln Leu Glu Glu

1715                    1720                    1725

Trp Leu  Arg Asp Lys Asn Leu  Met Asn Ser Gly Ala  Lys Glu Thr

1730                    1735                    1740

Leu Glu  Pro Leu Ile Gln Ala  Ala Gln Leu Leu Gln  Val Lys Lys

1745                    1750                    1755

Lys Thr  Asp Asp Asp Ala Glu  Ala Ile Cys Ser Met  Cys Asn Ala

1760                    1765                    1770

Leu Thr  Thr Ala Gln Ile Val  Lys Val Leu Asn Leu  Tyr Thr Pro

1775                    1780                    1785

Val Asn  Glu Phe Glu Glu Arg  Val Ser Val Ser Phe  Ile Arg Thr

1790                    1795                    1800

Ile Gln  Met Arg Leu Arg Asp  Arg Lys Asp Ser Pro  Gln Leu Leu

1805                    1810                    1815

Met Asp  Ala Lys His Ile Phe  Pro Val Thr Phe Pro  Phe Asn Pro

1820                    1825                    1830

Ser Ser  Leu Ala Leu Glu Thr  Ile Gln Ile Pro Ala  Ser Leu Gly

1835                    1840                    1845

Leu Gly  Phe Ile Ser Arg Val

1850                    1855.

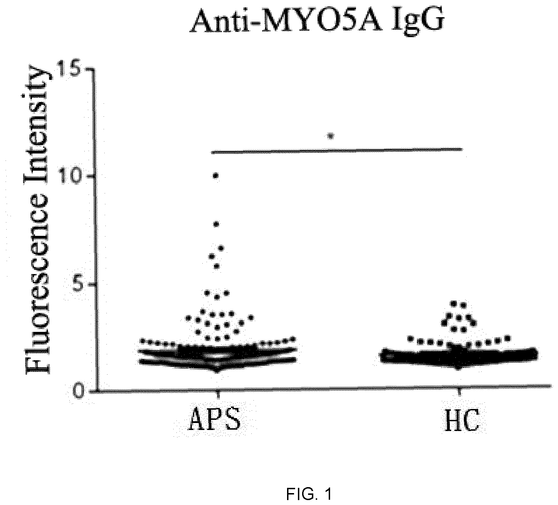

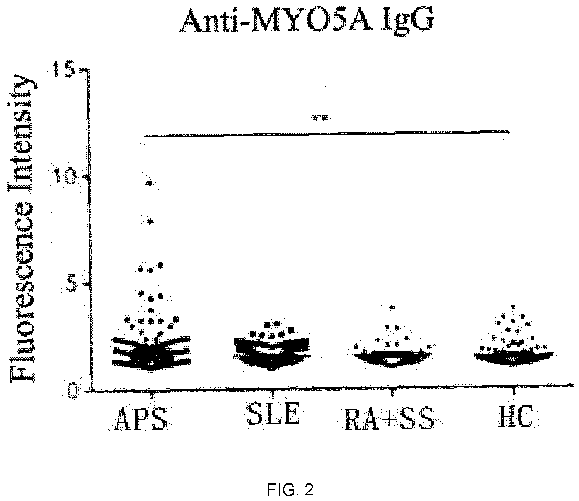

The above results are verified through experiments:
There is no significant difference between the antibodies to MYOSA protein in the APS group and in the disease control group, as shown in FIG. 1. The antibodies to MY05A protein only show a significant difference between the APS group and the healthy control group, as shown in Figure 2.

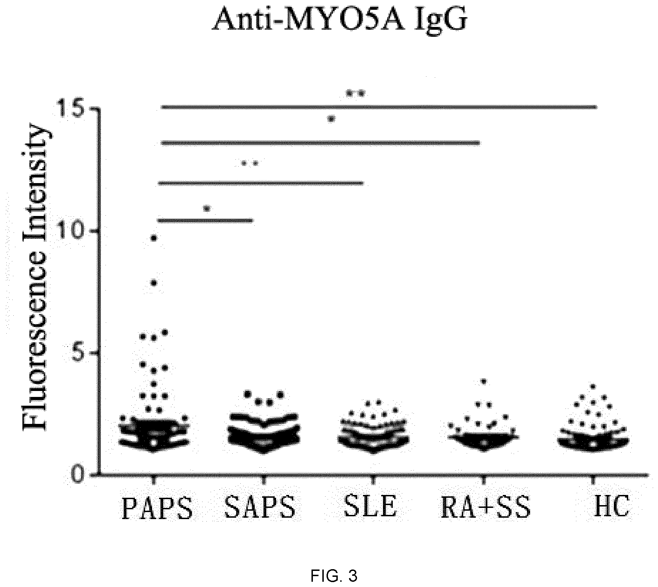

The APS group is divided into primary APS and secondary APS group in order to find differential protein antibodies between the subgroups, in which the results are shown in FIG. 3. The experiments find that the expression of antibodies to MYOSA protein exists a significant difference between the primary APS and secondary APS group, and also exists among the primary APS group, other disease control group and the healthy control group.

The present disclosure provides a new biomarker to identify antiphospholipid syndrome and a new application direction which is of great significance for the identification of antiphospholipid syndrome.

### Embodiment 2

The present embodiment provides the use of antibodies to MYOSA protein as a biomarker in the in vitro determination of antiphospholipid syndrome in biological samples selected from serum, plasma or tissue fluid obtained from a subject.

### Embodiment 3

The present embodiment provides the use of a reagent kit in the in vitro determination of antiphopholipid syndrome, the reagent kit comprising MYOSA protein, wherein the presence of antibodies to MYOSA protein in biological samples are detected, the biological samples being selected from serum, plasma or tissue fluid obtained from a subj ect.

Specifically, the reagent kit comprises MYOSA protein, and the detection method is any one of ELISA, chemiluminescence or POCT.

The reagent kit comprises MYOSA protein and the target molecule detected and identified by the reagent kit is the antibody produced by the MYOSA protein, including at least one of IgG, IgM or IgA.

## Claims

1. Use of antibodies to MYOSA protein as a biomarker in the *in vitro* determination of antiphospholipid syndrome in biological samples selected from serum, plasma or tissue fluid obtained from a subject.

2. Use of antibodies to MYOSA protein, according to claim 1, **characterized in that** the antiphospholipid syndrome is primary antiphospholipid syndrome.

3. Use of antibodies to MYOSA protein, according to any of claims 1 and 2, **characterized in that** the antiphospholipid syndrome is secondary antiphospholipid syndrome.

4. Use of antibodies to MYOSA protein, according to any of claims 1, 2 and 3, **characterized in that** primary antiphospholipid syndrome and secondary antiphospholipid syndrome are determined simultaneously.

5. Use of antibodies to MYOSA protein, according to any of claims 1, 2, 3 and 4, **characterized in that** the antibodies to MYOSA protein includes at least one of IgG, IgM or IgA.

6. Use of antibodies to MYOSA protein, according to any of claims 1, 2, 3, 4 or 5, **characterized in that** the sequence of MYOSA protein is,

Met Ala Ala Ser Glu Leu Tyr Thr Lys Phe Ala Arg Val Trp Ile Pro
1               5                   10                  15
Asp Pro Glu Glu Val Trp Lys Ser Ala Glu Leu Leu Lys Asp Tyr Lys
            20                  25                  30
Pro Gly Asp Lys Val Leu Leu Leu His Leu Glu Glu Gly Lys Asp Leu
        35                  40                  45
Glu Tyr His Leu Asp Pro Lys Thr Lys Glu Leu Pro His Leu Arg Asn
    50                  55                  60
Pro Asp Ile Leu Val Gly Glu Asn Asp Leu Thr Ala Leu Ser Tyr Leu

65                  70                  75                  80

His Glu Pro Ala Val Leu His Asn Leu Arg Val Arg Phe Ile Asp Ser

                85                  90                  95

Lys Leu Ile Tyr Thr Tyr Cys Gly Ile Val Leu Val Ala Ile Asn Pro

            100                 105                 110

Tyr Glu Gln Leu Pro Ile Tyr Gly Glu Asp Ile Ile Asn Ala Tyr Ser

        115                 120                 125

Gly Gln Asn Met Gly Asp Met Asp Pro His Ile Phe Ala Val Ala Glu

    130                 135                 140

Glu Ala Tyr Lys Gln Met Ala Arg Asp Glu Arg Asn Gln Ser Ile Ile

145                 150                 155                 160

Val Ser Gly Glu Ser Gly Ala Gly Lys Thr Val Ser Ala Lys Tyr Ala

                165                 170                 175

Met Arg Tyr Phe Ala Thr Val Ser Gly Ser Ala Ser Glu Ala Asn Val

            180                 185                 190

Glu Glu Lys Val Leu Ala Ser Asn Pro Ile Met Glu Ser Ile Gly Asn

        195                 200                 205

Ala Lys Thr Thr Arg Asn Asp Asn Ser Ser Arg Phe Gly Lys Tyr Ile

    210                 215                 220

Glu Ile Gly Phe Asp Lys Arg Tyr Arg Ile Ile Gly Ala Asn Met Arg

225                 230                 235                 240

Thr Tyr Leu Leu Glu Lys Ser Arg Val Val Phe Gln Ala Glu Glu Glu

                245                 250                 255

Arg Asn Tyr His Ile Phe Tyr Gln Leu Cys Ala Ser Ala Lys Leu Pro

            260                 265                 270

Glu Phe Lys Met Leu Arg Leu Gly Asn Ala Asp Asn Phe Asn Tyr Thr

        275                 280                 285

Lys Gln Gly Gly Ser Pro Val Ile Glu Gly Val Asp Asp Ala Lys Glu

    290                 295                 300

Met Ala His Thr Arg Gln Ala Cys Thr Leu Leu Gly Ile Ser Glu Ser
305                 310                 315                 320
His Gln Met Gly Ile Phe Arg Ile Leu Ala Gly Ile Leu His Leu Gly
                325                 330                 335
Asn Val Gly Phe Thr Ser Arg Asp Ala Asp Ser Cys Thr Ile Pro Pro
            340                 345                 350
Lys His Glu Pro Leu Cys Ile Phe Cys Glu Leu Met Gly Val Asp Tyr
        355                 360                 365
Glu Glu Met Cys His Trp Leu Cys His Arg Lys Leu Ala Thr Ala Thr
    370                 375                 380
Glu Thr Tyr Ile Lys Pro Ile Ser Lys Leu Gln Ala Thr Asn Ala Arg
385                 390                 395                 400
Asp Ala Leu Ala Lys His Ile Tyr Ala Lys Leu Phe Asn Trp Ile Val
                405                 410                 415
Asp Asn Val Asn Gln Ala Leu His Ser Ala Val Lys Gln His Ser Phe
            420                 425                 430
Ile Gly Val Leu Asp Ile Tyr Gly Phe Glu Thr Phe Glu Ile Asn Ser
        435                 440                 445
Phe Glu Gln Phe Cys Ile Asn Tyr Ala Asn Glu Lys Leu Gln Gln Gln
    450                 455                 460
Phe Asn Met His Val Phe Lys Leu Glu Gln Glu Glu Tyr Met Lys Glu
465                 470                 475                 480
Gln Ile Pro Trp Thr Leu Ile Asp Phe Tyr Asp Asn Gln Pro Cys Ile
                485                 490                 495
Asn Leu Ile Glu Ser Lys Leu Gly Ile Leu Asp Leu Leu Asp Glu Glu
            500                 505                 510
Cys Lys Met Pro Lys Gly Thr Asp Asp Thr Trp Ala Gln Lys Leu Tyr
        515                 520                 525
Asn Thr His Leu Asn Lys Cys Ala Leu Phe Glu Lys Pro Arg Leu Ser

    530                 535                 540

Asn Lys Ala Phe Ile Ile Gln His Phe Ala Asp Lys Val Glu Tyr Gln

545                 550                 555                 560

Cys Glu Gly Phe Leu Glu Lys Asn Lys Asp Thr Val Phe Glu Glu Gln

                565                 570                 575

Ile Lys Val Leu Lys Ser Ser Lys Phe Lys Met Leu Pro Glu Leu Phe

            580                 585                 590

Gln Asp Asp Glu Lys Ala Ile Ser Pro Thr Ser Ala Thr Ser Ser Gly

        595                 600                 605

Arg Thr Pro Leu Thr Arg Thr Pro Ala Lys Pro Thr Lys Gly Arg Pro

    610                 615                 620

Gly Gln Met Ala Lys Glu His Lys Lys Thr Val Gly His Gln Phe Arg

625                 630                 635                 640

Asn Ser Leu His Leu Leu Met Glu Thr Leu Asn Ala Thr Thr Pro His

                645                 650                 655

Tyr Val Arg Cys Ile Lys Pro Asn Asp Phe Lys Phe Pro Phe Thr Phe

            660                 665                 670

Asp Glu Lys Arg Ala Val Gln Gln Leu Arg Ala Cys Gly Val Leu Glu

        675                 680                 685

Thr Ile Arg Ile Ser Ala Ala Gly Phe Pro Ser Arg Trp Thr Tyr Gln

    690                 695                 700

Glu Phe Phe Ser Arg Tyr Arg Val Leu Met Lys Gln Lys Asp Val Leu

705                 710                 715                 720

Ser Asp Arg Lys Gln Thr Cys Lys Asn Val Leu Glu Lys Leu Ile Leu

                725                 730                 735

Asp Lys Asp Lys Tyr Gln Phe Gly Lys Thr Lys Ile Phe Phe Arg Ala

            740                 745                 750

Gly Gln Val Ala Tyr Leu Glu Lys Leu Arg Ala Asp Lys Leu Arg Ala

        755                 760                 765

Ala Cys Ile Arg Ile Gln Lys Thr Ile Arg Gly Trp Leu Leu Arg Lys
    770                 775                 780
Lys Tyr Leu Arg Met Arg Lys Ala Ala Ile Thr Met Gln Arg Tyr Val
785                 790                 795                 800
Arg Gly Tyr Gln Ala Arg Cys Tyr Ala Lys Phe Leu Arg Arg Thr Lys
                805                 810                 815
Ala Ala Thr Ile Ile Gln Lys Tyr Trp Arg Met Tyr Val Val Arg Arg
            820                 825                 830
Arg Tyr Lys Ile Arg Arg Ala Ala Thr Ile Val Leu Gln Ser Tyr Leu
        835                 840                 845
Arg Gly Phe Leu Ala Arg Asn Arg Tyr Arg Lys Ile Leu Arg Glu His
    850                 855                 860
Lys Ala Val Ile Ile Gln Lys Arg Val Arg Gly Trp Leu Ala Arg Thr
865                 870                 875                 880
His Tyr Lys Arg Ser Met His Ala Ile Ile Tyr Leu Gln Cys Cys Phe
                885                 890                 895
Arg Arg Met Met Ala Lys Arg Glu Leu Lys Lys Leu Lys Ile Glu Ala
            900                 905                 910
Arg Ser Val Glu Arg Tyr Lys Lys Leu His Ile Gly Met Glu Asn Lys
        915                 920                 925
Ile Met Gln Leu Gln Arg Lys Val Asp Glu Gln Asn Lys Asp Tyr Lys
    930                 935                 940
Cys Leu Val Glu Lys Leu Thr Asn Leu Glu Gly Ile Tyr Asn Ser Glu
945                 950                 955                 960
Thr Glu Lys Leu Arg Ser Asp Leu Glu Arg Leu Gln Leu Ser Glu Glu
                965                 970                 975
Glu Ala Lys Val Ala Thr Gly Arg Val Leu Ser Leu Gln Glu Glu Ile
            980                 985                 990
Ala Lys Leu Arg Lys Asp Leu Glu Gln Thr Arg Ser Glu Lys Lys Cys

995                 1000                1005
Ile Glu  Glu His Ala Asp Arg  Tyr Lys Gln Glu Thr  Glu Gln Leu
    1010                1015                1020
Val Ser  Asn Leu Lys Glu Glu  Asn Thr Leu Leu Lys  Gln Glu Lys
    1025                1030                1035
Glu Ala  Leu Asn His Arg Ile  Val Gln Gln Ala Lys  Glu Met Thr
    1040                1045                1050
Glu Thr  Met Glu Lys Lys Leu  Val Glu Glu Thr Lys  Gln Leu Glu
    1055                1060                1065
Leu Asp  Leu Asn Asp Glu Arg  Leu Arg Tyr Gln Asn  Leu Leu Asn
    1070                1075                1080
Glu Phe  Ser Arg Leu Glu Glu  Arg Tyr Asp Asp Leu  Lys Glu Glu
    1085                1090                1095
Met Thr  Leu Met Val His Val  Pro Lys Pro Gly His  Lys Arg Thr
    1100                1105                1110
Asp Ser  Thr His Ser Ser Asn  Glu Ser Glu Tyr Ile  Phe Ser Ser
    1115                1120                1125
Glu Ile  Ala Glu Met Glu Asp  Ile Pro Ser Arg Thr  Glu Glu Pro
    1130                1135                1140
Ser Glu  Lys Lys Val Pro Leu  Asp Met Ser Leu Phe  Leu Lys Leu
    1145                1150                1155
Gln Lys  Arg Val Thr Glu Leu  Glu Gln Glu Lys Gln  Val Met Gln
    1160                1165                1170
Asp Glu  Leu Asp Arg Lys Glu  Glu Gln Val Leu Arg  Ser Lys Ala
    1175                1180                1185
Lys Glu  Glu Glu Arg Pro Gln  Ile Arg Gly Ala Glu  Leu Glu Tyr
    1190                1195                1200
Glu Ser  Leu Lys Arg Gln Glu  Leu Glu Ser Glu Asn  Lys Lys Leu
    1205                1210                1215

Lys Asn Glu Leu Asn Glu Leu Arg Lys Ala Leu Ser Glu Lys Ser
    1220                1225                1230
Ala Pro Glu Val Thr Ala Pro Gly Ala Pro Ala Tyr Arg Val Leu
    1235                1240                1245
Met Glu Gln Leu Thr Ser Val Ser Glu Glu Leu Asp Val Arg Lys
    1250                1255                1260
Glu Glu Val Leu Ile Leu Arg Ser Gln Leu Val Ser Gln Lys Glu
    1265                1270                1275
Ala Ile Gln Pro Lys Asp Asp Lys Asn Thr Met Thr Asp Ser Thr
    1280                1285                1290
Ile Leu Leu Glu Asp Val Gln Lys Met Lys Asp Lys Gly Glu Ile
    1295                1300                1305
Ala Gln Ala Tyr Ile Gly Leu Lys Glu Thr Asn Arg Ser Ser Ala
    1310                1315                1320
Leu Asp Tyr His Glu Leu Asn Glu Asp Gly Glu Leu Trp Leu Val
    1325                1330                1335
Tyr Glu Gly Leu Lys Gln Ala Asn Arg Leu Leu Glu Ser Gln Leu
    1340                1345                1350
Gln Ser Gln Lys Arg Ser His Glu Asn Glu Ala Glu Ala Leu Arg
    1355                1360                1365
Gly Glu Ile Gln Ser Leu Lys Glu Glu Asn Asn Arg Gln Gln Gln
    1370                1375                1380
Leu Leu Ala Gln Asn Leu Gln Leu Pro Pro Glu Ala Arg Ile Glu
    1385                1390                1395
Ala Ser Leu Gln His Glu Ile Thr Arg Leu Thr Asn Glu Asn Leu
    1400                1405                1410
Asp Leu Met Glu Gln Leu Glu Lys Gln Asp Lys Thr Val Arg Lys
    1415                1420                1425
Leu Lys Lys Gln Leu Lys Val Phe Ala Lys Lys Ile Gly Glu Leu

1430                    1435                    1440

Glu Val  Gly Gln Met Glu Asn  Ile Ser Pro Gly Gln  Ile Ile Asp

1445                    1450                    1455

Glu Pro  Ile Arg Pro Val Asn  Ile Pro Arg Lys Glu  Lys Asp Phe

1460                    1465                    1470

Gln Gly  Met Leu Glu Tyr Lys  Lys Glu Asp Glu Gln  Lys Leu Val

1475                    1480                    1485

Lys Asn  Leu Ile Leu Glu Leu  Lys Pro Arg Gly Val  Ala Val Asn

1490                    1495                    1500

Leu Ile  Pro Gly Leu Pro Ala  Tyr Ile Leu Phe Met  Cys Val Arg

1505                    1510                    1515

His Ala  Asp Tyr Leu Asn Asp  Asp Gln Lys Val Arg  Ser Leu Leu

1520                    1525                    1530

Thr Ser  Thr Ile Asn Ser Ile  Lys Lys Val Leu Lys  Lys Arg Gly

1535                    1540                    1545

Asp Asp  Phe Glu Thr Val Ser  Phe Trp Leu Ser Asn  Thr Cys Arg

1550                    1555                    1560

Phe Leu  His Cys Leu Lys Gln  Tyr Ser Gly Glu Glu  Gly Phe Met

1565                    1570                    1575

Lys His  Asn Thr Ser Arg Gln  Asn Glu His Cys Leu  Thr Asn Phe

1580                    1585                    1590

Asp Leu  Ala Glu Tyr Arg Gln  Val Leu Ser Asp Leu  Ala Ile Gln

1595                    1600                    1605

Ile Tyr  Gln Gln Leu Val Arg  Val Leu Glu Asn Ile  Leu Gln Pro

1610                    1615                    1620

Met Ile  Val Ser Gly Met Leu  Glu His Glu Thr Ile  Gln Gly Val

1625                    1630                    1635

Ser Gly  Val Lys Pro Thr Gly  Leu Arg Lys Arg Thr  Ser Ser Ile

1640                    1645                    1650

Ala Asp Glu Gly Thr Tyr Thr Leu Asp Ser Ile Leu Arg Gln Leu
    1655                1660                1665
Asn Ser Phe His Ser Val Met Cys Gln His Gly Met Asp Pro Glu
    1670                1675                1680
Leu Ile Lys Gln Val Val Lys Gln Met Phe Tyr Ile Ile Gly Ala
    1685                1690                1695
Ile Thr Leu Asn Asn Leu Leu Leu Arg Lys Asp Met Cys Ser Trp
    1700                1705                1710
Ser Lys Gly Met Gln Ile Arg Tyr Asn Val Ser Gln Leu Glu Glu
    1715                1720                1725
Trp Leu Arg Asp Lys Asn Leu Met Asn Ser Gly Ala Lys Glu Thr
    1730                1735                1740
Leu Glu Pro Leu Ile Gln Ala Ala Gln Leu Leu Gln Val Lys Lys
    1745                1750                1755
Lys Thr Asp Asp Asp Ala Glu Ala Ile Cys Ser Met Cys Asn Ala
    1760                1765                1770
Leu Thr Thr Ala Gln Ile Val Lys Val Leu Asn Leu Tyr Thr Pro
    1775                1780                1785
Val Asn Glu Phe Glu Glu Arg Val Ser Val Ser Phe Ile Arg Thr
    1790                1795                1800
Ile Gln Met Arg Leu Arg Asp Arg Lys Asp Ser Pro Gln Leu Leu
    1805                1810                1815
Met Asp Ala Lys His Ile Phe Pro Val Thr Phe Pro Phe Asn Pro
    1820                1825                1830
Ser Ser Leu Ala Leu Glu Thr Ile Gln Ile Pro Ala Ser Leu Gly
    1835                1840                1845
Leu Gly Phe Ile Ser Arg Val
1850 1855 .

7. Use of a reagent kit in the *in vitro* determination of antiphopholipid syndrome **characterized in that** the reagent kit comprises MYOSA protein, wherein the presence of antibodies to MYOSA protein in biological samples are detected, the biological samples being selected from serum, plasma or tissue fluid obtained from a subject.

8. Use of a reagent kit in the *in vitro* determination of antiphopholipid syndrome, according to claim 7, **characterized in that** the antibodies to MYOSA protein include at least one of IgG, IgM or IgA.

9. Use of a reagent kit in the *in vitro* determination of antiphopholipid syndrome, according to any of claims 7 and 8, **characterized in that** the antibodies to MYOSA protein are detected by a detection method selected from any one of ELISA, chemiluminescence or POCT.

## Patentansprüche

1. Verwendung von Antikörpern gegen das MYOSA-Protein **dadurch gekennzeichnet, dass** es als Biomarker bei der *in vitro* Bestimmung des Antiphospholipid-Syndroms in biologischen Proben verwendet wird, die aus Serum, Plasma oder Gewebeflüssigkeit ausgewählt und von einem Probanden erhalten wurden.

2. Verwendung von Antikörpern gegen das MYOSA-Protein gemäß Anspruch 1, **dadurch gekennzeichnet, dass** das Antiphospholipid-Syndrom ein primäres Antiphospholipid-Syndrom ist.

3. Verwendung von Antikörpern gegen das MYOSA-Protein gemäß einem der Ansprüche 1 und 2, **dadurch gekennzeichnet, dass** das Antiphospholipid-Syndrom ein sekundäres Antiphospholipid-Syndrom ist.

4. Verwendung von Antikörpern gegen das MYOSA-Protein gemäß einem der Ansprüche 1, 2 und 3, **dadurch gekennzeichnet, dass** das primäre Antiphospholipid-Syndrom und das sekundäre Antiphospholipid-Syndrom gleichzeitig bestimmt werden.

5. Verwendung von Antikörpern gegen das MYOSA-Protein gemäß einem der Ansprüche 1, 2, 3 und 4, **dadurch gekennzeichnet, dass** die Antikörper gegen das MYOSA-Protein mindestens ein IgG, IgM oder IgA enthalten.

6. Verwendung von Antikörpern gegen das MYOSA-Protein gemäß einem der Ansprüche 1, 2, 3, 4 oder 5, **dadurch gekennzeichnet, dass** das MYOSA-Protein folgende Sequenz aufweist,

Met Ala Ala Ser Glu Leu Tyr Thr Lys Phe Ala Arg Val Trp Ile Pro
1               5                   10                  15
Asp Pro Glu Glu Val Trp Lys Ser Ala Glu Leu Leu Lys Asp Tyr Lys
            20                  25                  30
Pro Gly Asp Lys Val Leu Leu Leu His Leu Glu Glu Gly Lys Asp Leu
        35                  40                  45

Glu Tyr His Leu Asp Pro Lys Thr Lys Glu Leu Pro His Leu Arg Asn
    50                  55                  60
Pro Asp Ile Leu Val Gly Glu Asn Asp Leu Thr Ala Leu Ser Tyr Leu
65                  70                  75                  80
His Glu Pro Ala Val Leu His Asn Leu Arg Val Arg Phe Ile Asp Ser
                85                  90                  95
Lys Leu Ile Tyr Thr Tyr Cys Gly Ile Val Leu Val Ala Ile Asn Pro
            100                 105                 110
Tyr Glu Gln Leu Pro Ile Tyr Gly Glu Asp Ile Ile Asn Ala Tyr Ser
        115                 120                 125
Gly Gln Asn Met Gly Asp Met Asp Pro His Ile Phe Ala Val Ala Glu
    130                 135                 140
Glu Ala Tyr Lys Gln Met Ala Arg Asp Glu Arg Asn Gln Ser Ile Ile
145                 150                 155                 160
Val Ser Gly Glu Ser Gly Ala Gly Lys Thr Val Ser Ala Lys Tyr Ala
                165                 170                 175
Met Arg Tyr Phe Ala Thr Val Ser Gly Ser Ala Ser Glu Ala Asn Val
            180                 185                 190
Glu Glu Lys Val Leu Ala Ser Asn Pro Ile Met Glu Ser Ile Gly Asn
        195                 200                 205
Ala Lys Thr Thr Arg Asn Asp Asn Ser Ser Arg Phe Gly Lys Tyr Ile
    210                 215                 220
Glu Ile Gly Phe Asp Lys Arg Tyr Arg Ile Ile Gly Ala Asn Met Arg
225                 230                 235                 240
Thr Tyr Leu Leu Glu Lys Ser Arg Val Val Phe Gln Ala Glu Glu Glu
                245                 250                 255
Arg Asn Tyr His Ile Phe Tyr Gln Leu Cys Ala Ser Ala Lys Leu Pro
            260                 265                 270
Glu Phe Lys Met Leu Arg Leu Gly Asn Ala Asp Asn Phe Asn Tyr Thr
        275                 280                 285
Lys Gln Gly Gly Ser Pro Val Ile Glu Gly Val Asp Asp Ala Lys Glu
    290                 295                 300

Met Ala His Thr Arg Gln Ala Cys Thr Leu Leu Gly Ile Ser Glu Ser
305                 310                 315                 320
His Gln Met Gly Ile Phe Arg Ile Leu Ala Gly Ile Leu His Leu Gly
                325                 330                 335
Asn Val Gly Phe Thr Ser Arg Asp Ala Asp Ser Cys Thr Ile Pro Pro
            340                 345                 350
Lys His Glu Pro Leu Cys Ile Phe Cys Glu Leu Met Gly Val Asp Tyr
        355                 360                 365
Glu Glu Met Cys His Trp Leu Cys His Arg Lys Leu Ala Thr Ala Thr
    370                 375                 380
Glu Thr Tyr Ile Lys Pro Ile Ser Lys Leu Gln Ala Thr Asn Ala Arg
385                 390                 395                 400
Asp Ala Leu Ala Lys His Ile Tyr Ala Lys Leu Phe Asn Trp Ile Val
                405                 410                 415
Asp Asn Val Asn Gln Ala Leu His Ser Ala Val Lys Gln His Ser Phe
            420                 425                 430
Ile Gly Val Leu Asp Ile Tyr Gly Phe Glu Thr Phe Glu Ile Asn Ser
        435                 440                 445
Phe Glu Gln Phe Cys Ile Asn Tyr Ala Asn Glu Lys Leu Gln Gln Gln
    450                 455                 460
Phe Asn Met His Val Phe Lys Leu Glu Gln Glu Glu Tyr Met Lys Glu
465                 470                 475                 480
Gln Ile Pro Trp Thr Leu Ile Asp Phe Tyr Asp Asn Gln Pro Cys Ile
                485                 490                 495
Asn Leu Ile Glu Ser Lys Leu Gly Ile Leu Asp Leu Leu Asp Glu Glu
            500                 505                 510
Cys Lys Met Pro Lys Gly Thr Asp Asp Thr Trp Ala Gln Lys Leu Tyr
        515                 520                 525
Asn Thr His Leu Asn Lys Cys Ala Leu Phe Glu Lys Pro Arg Leu Ser

    530                 535                 540

Asn Lys Ala Phe Ile Ile Gln His Phe Ala Asp Lys Val Glu Tyr Gln

545                 550                 555                 560

Cys Glu Gly Phe Leu Glu Lys Asn Lys Asp Thr Val Phe Glu Glu Gln

                565                 570                 575

Ile Lys Val Leu Lys Ser Ser Lys Phe Lys Met Leu Pro Glu Leu Phe

            580                 585                 590

Gln Asp Asp Glu Lys Ala Ile Ser Pro Thr Ser Ala Thr Ser Ser Gly

        595                 600                 605

Arg Thr Pro Leu Thr Arg Thr Pro Ala Lys Pro Thr Lys Gly Arg Pro

    610                 615                 620

Gly Gln Met Ala Lys Glu His Lys Lys Thr Val Gly His Gln Phe Arg

625                 630                 635                 640

Asn Ser Leu His Leu Leu Met Glu Thr Leu Asn Ala Thr Thr Pro His

                645                 650                 655

Tyr Val Arg Cys Ile Lys Pro Asn Asp Phe Lys Phe Pro Phe Thr Phe

            660                 665                 670

Asp Glu Lys Arg Ala Val Gln Gln Leu Arg Ala Cys Gly Val Leu Glu

        675                 680                 685

Thr Ile Arg Ile Ser Ala Ala Gly Phe Pro Ser Arg Trp Thr Tyr Gln

    690                 695                 700

Glu Phe Phe Ser Arg Tyr Arg Val Leu Met Lys Gln Lys Asp Val Leu

705                 710                 715                 720

Ser Asp Arg Lys Gln Thr Cys Lys Asn Val Leu Glu Lys Leu Ile Leu

                725                 730                 735

Asp Lys Asp Lys Tyr Gln Phe Gly Lys Thr Lys Ile Phe Phe Arg Ala

            740                 745                 750

Gly Gln Val Ala Tyr Leu Glu Lys Leu Arg Ala Asp Lys Leu Arg Ala

        755                 760                 765

Ala Cys Ile Arg Ile Gln Lys Thr Ile Arg Gly Trp Leu Leu Arg Lys
    770                 775                 780
Lys Tyr Leu Arg Met Arg Lys Ala Ala Ile Thr Met Gln Arg Tyr Val
785                 790                 795                 800
Arg Gly Tyr Gln Ala Arg Cys Tyr Ala Lys Phe Leu Arg Arg Thr Lys
                805                 810                 815
Ala Ala Thr Ile Ile Gln Lys Tyr Trp Arg Met Tyr Val Val Arg Arg
            820                 825                 830
Arg Tyr Lys Ile Arg Arg Ala Ala Thr Ile Val Leu Gln Ser Tyr Leu
        835                 840                 845
Arg Gly Phe Leu Ala Arg Asn Arg Tyr Arg Lys Ile Leu Arg Glu His
    850                 855                 860
Lys Ala Val Ile Ile Gln Lys Arg Val Arg Gly Trp Leu Ala Arg Thr
865                 870                 875                 880
His Tyr Lys Arg Ser Met His Ala Ile Ile Tyr Leu Gln Cys Cys Phe
                885                 890                 895
Arg Arg Met Met Ala Lys Arg Glu Leu Lys Lys Leu Lys Ile Glu Ala
            900                 905                 910
Arg Ser Val Glu Arg Tyr Lys Lys Leu His Ile Gly Met Glu Asn Lys
        915                 920                 925
Ile Met Gln Leu Gln Arg Lys Val Asp Glu Gln Asn Lys Asp Tyr Lys
    930                 935                 940
Cys Leu Val Glu Lys Leu Thr Asn Leu Glu Gly Ile Tyr Asn Ser Glu
945                 950                 955                 960
Thr Glu Lys Leu Arg Ser Asp Leu Glu Arg Leu Gln Leu Ser Glu Glu
                965                 970                 975
Glu Ala Lys Val Ala Thr Gly Arg Val Leu Ser Leu Gln Glu Glu Ile
            980                 985                 990
Ala Lys Leu Arg Lys Asp Leu Glu Gln Thr Arg Ser Glu Lys Lys Cys

        995                 1000                1005
Ile Glu Glu His Ala Asp Arg Tyr Lys Gln Glu Thr Glu Gln Leu
    1010                1015                1020
Val Ser Asn Leu Lys Glu Glu Asn Thr Leu Leu Lys Gln Glu Lys
    1025                1030                1035
Glu Ala Leu Asn His Arg Ile Val Gln Gln Ala Lys Glu Met Thr
    1040                1045                1050
Glu Thr Met Glu Lys Lys Leu Val Glu Glu Thr Lys Gln Leu Glu
    1055                1060                1065
Leu Asp Leu Asn Asp Glu Arg Leu Arg Tyr Gln Asn Leu Leu Asn
    1070                1075                1080
Glu Phe Ser Arg Leu Glu Glu Arg Tyr Asp Asp Leu Lys Glu Glu
    1085                1090                1095
Met Thr Leu Met Val His Val Pro Lys Pro Gly His Lys Arg Thr
    1100                1105                1110
Asp Ser Thr His Ser Ser Asn Glu Ser Glu Tyr Ile Phe Ser Ser
    1115                1120                1125
Glu Ile Ala Glu Met Glu Asp Ile Pro Ser Arg Thr Glu Glu Pro
    1130                1135                1140
Ser Glu Lys Lys Val Pro Leu Asp Met Ser Leu Phe Leu Lys Leu
    1145                1150                1155
Gln Lys Arg Val Thr Glu Leu Glu Gln Glu Lys Gln Val Met Gln
    1160                1165                1170
Asp Glu Leu Asp Arg Lys Glu Glu Gln Val Leu Arg Ser Lys Ala
    1175                1180                1185
Lys Glu Glu Glu Arg Pro Gln Ile Arg Gly Ala Glu Leu Glu Tyr
    1190                1195                1200
Glu Ser Leu Lys Arg Gln Glu Leu Glu Ser Glu Asn Lys Lys Leu
    1205                1210                1215

Lys Asn Glu Leu Asn Glu Leu Arg Lys Ala Leu Ser Glu Lys Ser
    1220                1225                1230
Ala Pro Glu Val Thr Ala Pro Gly Ala Pro Ala Tyr Arg Val Leu
    1235                1240                1245
Met Glu Gln Leu Thr Ser Val Ser Glu Glu Leu Asp Val Arg Lys
    1250                1255                1260
Glu Glu Val Leu Ile Leu Arg Ser Gln Leu Val Ser Gln Lys Glu
    1265                1270                1275
Ala Ile Gln Pro Lys Asp Asp Lys Asn Thr Met Thr Asp Ser Thr
    1280                1285                1290
Ile Leu Leu Glu Asp Val Gln Lys Met Lys Asp Lys Gly Glu Ile
    1295                1300                1305
Ala Gln Ala Tyr Ile Gly Leu Lys Glu Thr Asn Arg Ser Ser Ala
    1310                1315                1320
Leu Asp Tyr His Glu Leu Asn Glu Asp Gly Glu Leu Trp Leu Val
    1325                1330                1335
Tyr Glu Gly Leu Lys Gln Ala Asn Arg Leu Leu Glu Ser Gln Leu
    1340                1345                1350
Gln Ser Gln Lys Arg Ser His Glu Asn Glu Ala Glu Ala Leu Arg
    1355                1360                1365
Gly Glu Ile Gln Ser Leu Lys Glu Glu Asn Asn Arg Gln Gln Gln
    1370                1375                1380
Leu Leu Ala Gln Asn Leu Gln Leu Pro Pro Glu Ala Arg Ile Glu
    1385                1390                1395
Ala Ser Leu Gln His Glu Ile Thr Arg Leu Thr Asn Glu Asn Leu
    1400                1405                1410
Asp Leu Met Glu Gln Leu Glu Lys Gln Asp Lys Thr Val Arg Lys
    1415                1420                1425
Leu Lys Lys Gln Leu Lys Val Phe Ala Lys Lys Ile Gly Glu Leu

1430                    1435                    1440

Glu Val  Gly Gln Met Glu Asn  Ile Ser Pro Gly Gln  Ile Ile Asp

1445                    1450                    1455

Glu Pro  Ile Arg Pro Val Asn  Ile Pro Arg Lys Glu  Lys Asp Phe

1460                    1465                    1470

Gln Gly  Met Leu Glu Tyr Lys  Lys Glu Asp Glu Gln  Lys Leu Val

1475                    1480                    1485

Lys Asn  Leu Ile Leu Glu Leu  Lys Pro Arg Gly Val  Ala Val Asn

1490                    1495                    1500

Leu Ile  Pro Gly Leu Pro Ala  Tyr Ile Leu Phe Met  Cys Val Arg

1505                    1510                    1515

His Ala  Asp Tyr Leu Asn Asp  Asp Gln Lys Val Arg  Ser Leu Leu

1520                    1525                    1530

Thr Ser  Thr Ile Asn Ser Ile  Lys Lys Val Leu Lys  Lys Arg Gly

1535                    1540                    1545

Asp Asp  Phe Glu Thr Val Ser  Phe Trp Leu Ser Asn  Thr Cys Arg

1550                    1555                    1560

Phe Leu  His Cys Leu Lys Gln  Tyr Ser Gly Glu Glu  Gly Phe Met

1565                    1570                    1575

Lys His  Asn Thr Ser Arg Gln  Asn Glu His Cys Leu  Thr Asn Phe

1580                    1585                    1590

Asp Leu  Ala Glu Tyr Arg Gln  Val Leu Ser Asp Leu  Ala Ile Gln

1595                    1600                    1605

Ile Tyr  Gln Gln Leu Val Arg  Val Leu Glu Asn Ile  Leu Gln Pro

1610                    1615                    1620

Met Ile  Val Ser Gly Met Leu  Glu His Glu Thr Ile  Gln Gly Val

1625                    1630                    1635

Ser Gly  Val Lys Pro Thr Gly  Leu Arg Lys Arg Thr  Ser Ser Ile

1640                    1645                    1650

Ala Asp Glu Gly Thr Tyr Thr Leu Asp Ser Ile Leu Arg Gln Leu
    1655                1660                1665
Asn Ser Phe His Ser Val Met Cys Gln His Gly Met Asp Pro Glu
    1670                1675                1680
Leu Ile Lys Gln Val Val Lys Gln Met Phe Tyr Ile Ile Gly Ala
    1685                1690                1695
Ile Thr Leu Asn Asn Leu Leu Leu Arg Lys Asp Met Cys Ser Trp
    1700                1705                1710
Ser Lys Gly Met Gln Ile Arg Tyr Asn Val Ser Gln Leu Glu Glu
    1715                1720                1725
Trp Leu Arg Asp Lys Asn Leu Met Asn Ser Gly Ala Lys Glu Thr
    1730                1735                1740
Leu Glu Pro Leu Ile Gln Ala Ala Gln Leu Leu Gln Val Lys Lys
    1745                1750                1755
Lys Thr Asp Asp Asp Ala Glu Ala Ile Cys Ser Met Cys Asn Ala
    1760                1765                1770
Leu Thr Thr Ala Gln Ile Val Lys Val Leu Asn Leu Tyr Thr Pro
    1775                1780                1785
Val Asn Glu Phe Glu Glu Arg Val Ser Val Ser Phe Ile Arg Thr
    1790                1795                1800
Ile Gln Met Arg Leu Arg Asp Arg Lys Asp Ser Pro Gln Leu Leu
    1805                1810                1815
Met Asp Ala Lys His Ile Phe Pro Val Thr Phe Pro Phe Asn Pro
    1820                1825                1830
Ser Ser Leu Ala Leu Glu Thr Ile Gln Ile Pro Ala Ser Leu Gly
    1835                1840                1845
Leu Gly Phe Ile Ser Arg Val
1850 1855 .

7. Verwendung eines Reagenzienkits bei der *in-vitro* -Bestimmung des Antiphopholipid-Syndroms **dadurch gekennzeichnet, dass** das Reagenzienkit MYOSA-Protein enthält, worin das Vorhandensein von Antikörpern gegen das MYOSA-Protein in biologischen Proben nachgewiesen wird, wobei die biologischen Proben aus Serum, Plasma oder Gewebeflüssigkeit eines Probanden ausgewählt werden.

8. Verwendung eines Reagenzienkits bei der *in-vitro* -Bestimmung des Antiphopholipid-Syndroms gemäß Anspruch 7, **dadurch gekennzeichnet, dass** die Antikörper gegen das MYOSA-Protein mindestens ein IgG, IgM oder IgA enthalten.

9. Verwendung eines Reagenzienkits bei der *in-vitro* -Bestimmung des Antiphopholipid-Syndroms gemäß einem der Ansprüche 7 und 8, **dadurch gekennzeichnet, dass** die Antikörper gegen das MYOSA-Protein durch ein aus ELISA, Chemolumineszenz oder POCT ausgewähltes Nachweisverfahren nachgewiesen werden.

## Revendications

1. Utilisation d'anticorps contre la protéine MYOSA **caractérisée en ce qu'**il s'agit d'un biomarqueur dans la détermination *in vitro* du syndrome des antiphospholipides dans des échantillons biologiques sélectionnés parmi du sérum, du plasma ou du liquide tissulaire obtenu à partir d'un sujet.

2. Utilisation d'anticorps contre la protéine MYOSA, selon la revendication 1, **caractérisée en ce que** le syndrome des antiphospholipides est un syndrome des antiphospholipides primaire.

3. Utilisation d'anticorps contre la protéine MYOSA, selon l'une quelconque des revendications 1 et 2, **caractérisée en ce que** le syndrome des antiphospholipides est un syndrome des antiphospholipides secondaire.

4. Utilisation d'anticorps contre la protéine MYOSA, selon l'une quelconque des revendications 1, 2 et 3, **caractérisée en ce que** le syndrome des antiphospholipides primaire et le syndrome des antiphospholipides secondaire sont déterminés simultanément.

5. Utilisation d'anticorps contre la protéine MYOSA, selon l'une quelconque des revendications 1, 2, 3 et 4, **caractérisée en ce que** les anticorps contre la protéine MYOSA comprennent au moins une parmi IgG, IgM ou IgA.

6. Utilisation d'anticorps contre la protéine MYOSA, selon l'une quelconque des revendications 1, 2, 3, 4 ou 5, **caractérisée en ce que** la séquence de la protéine MYOSA est,

Met Ala Ala Ser Glu Leu Tyr Thr Lys Phe Ala Arg Val Trp Ile Pro
1               5                   10                  15
Asp Pro Glu Glu Val Trp Lys Ser Ala Glu Leu Leu Lys Asp Tyr Lys
            20                  25                  30
Pro Gly Asp Lys Val Leu Leu Leu His Leu Glu Glu Gly Lys Asp Leu
        35                  40                  45
Glu Tyr His Leu Asp Pro Lys Thr Lys Glu Leu Pro His Leu Arg Asn
    50                  55                  60
Pro Asp Ile Leu Val Gly Glu Asn Asp Leu Thr Ala Leu Ser Tyr Leu

65                  70                  75                  80

His Glu Pro Ala Val Leu His Asn Leu Arg Val Arg Phe Ile Asp Ser

                85                  90                  95

Lys Leu Ile Tyr Thr Tyr Cys Gly Ile Val Leu Val Ala Ile Asn Pro

            100                 105                 110

Tyr Glu Gln Leu Pro Ile Tyr Gly Glu Asp Ile Ile Asn Ala Tyr Ser

        115                 120                 125

Gly Gln Asn Met Gly Asp Met Asp Pro His Ile Phe Ala Val Ala Glu

    130                 135                 140

Glu Ala Tyr Lys Gln Met Ala Arg Asp Glu Arg Asn Gln Ser Ile Ile

145                 150                 155                 160

Val Ser Gly Glu Ser Gly Ala Gly Lys Thr Val Ser Ala Lys Tyr Ala

                165                 170                 175

Met Arg Tyr Phe Ala Thr Val Ser Gly Ser Ala Ser Glu Ala Asn Val

            180                 185                 190

Glu Glu Lys Val Leu Ala Ser Asn Pro Ile Met Glu Ser Ile Gly Asn

        195                 200                 205

Ala Lys Thr Thr Arg Asn Asp Asn Ser Ser Arg Phe Gly Lys Tyr Ile

    210                 215                 220

Glu Ile Gly Phe Asp Lys Arg Tyr Arg Ile Ile Gly Ala Asn Met Arg

225                 230                 235                 240

Thr Tyr Leu Leu Glu Lys Ser Arg Val Val Phe Gln Ala Glu Glu Glu

                245                 250                 255

Arg Asn Tyr His Ile Phe Tyr Gln Leu Cys Ala Ser Ala Lys Leu Pro

            260                 265                 270

Glu Phe Lys Met Leu Arg Leu Gly Asn Ala Asp Asn Phe Asn Tyr Thr

        275                 280                 285

Lys Gln Gly Gly Ser Pro Val Ile Glu Gly Val Asp Asp Ala Lys Glu

    290                 295                 300

Met Ala His Thr Arg Gln Ala Cys Thr Leu Leu Gly Ile Ser Glu Ser
305                 310                 315                 320
His Gln Met Gly Ile Phe Arg Ile Leu Ala Gly Ile Leu His Leu Gly
                325                 330                 335
Asn Val Gly Phe Thr Ser Arg Asp Ala Asp Ser Cys Thr Ile Pro Pro
            340                 345                 350
Lys His Glu Pro Leu Cys Ile Phe Cys Glu Leu Met Gly Val Asp Tyr
        355                 360                 365
Glu Glu Met Cys His Trp Leu Cys His Arg Lys Leu Ala Thr Ala Thr
    370                 375                 380
Glu Thr Tyr Ile Lys Pro Ile Ser Lys Leu Gln Ala Thr Asn Ala Arg
385                 390                 395                 400
Asp Ala Leu Ala Lys His Ile Tyr Ala Lys Leu Phe Asn Trp Ile Val
                405                 410                 415
Asp Asn Val Asn Gln Ala Leu His Ser Ala Val Lys Gln His Ser Phe
            420                 425                 430
Ile Gly Val Leu Asp Ile Tyr Gly Phe Glu Thr Phe Glu Ile Asn Ser
        435                 440                 445
Phe Glu Gln Phe Cys Ile Asn Tyr Ala Asn Glu Lys Leu Gln Gln Gln
    450                 455                 460
Phe Asn Met His Val Phe Lys Leu Glu Gln Glu Glu Tyr Met Lys Glu
465                 470                 475                 480
Gln Ile Pro Trp Thr Leu Ile Asp Phe Tyr Asp Asn Gln Pro Cys Ile
                485                 490                 495
Asn Leu Ile Glu Ser Lys Leu Gly Ile Leu Asp Leu Leu Asp Glu Glu
            500                 505                 510
Cys Lys Met Pro Lys Gly Thr Asp Asp Thr Trp Ala Gln Lys Leu Tyr
        515                 520                 525
Asn Thr His Leu Asn Lys Cys Ala Leu Phe Glu Lys Pro Arg Leu Ser

    530                 535                 540
Asn Lys Ala Phe Ile Ile Gln His Phe Ala Asp Lys Val Glu Tyr Gln
545                 550                 555                 560
Cys Glu Gly Phe Leu Glu Lys Asn Lys Asp Thr Val Phe Glu Glu Gln
                565                 570                 575
Ile Lys Val Leu Lys Ser Ser Lys Phe Lys Met Leu Pro Glu Leu Phe
            580                 585                 590
Gln Asp Asp Glu Lys Ala Ile Ser Pro Thr Ser Ala Thr Ser Ser Gly
        595                 600                 605
Arg Thr Pro Leu Thr Arg Thr Pro Ala Lys Pro Thr Lys Gly Arg Pro
    610                 615                 620
Gly Gln Met Ala Lys Glu His Lys Lys Thr Val Gly His Gln Phe Arg
625                 630                 635                 640
Asn Ser Leu His Leu Leu Met Glu Thr Leu Asn Ala Thr Thr Pro His
                645                 650                 655
Tyr Val Arg Cys Ile Lys Pro Asn Asp Phe Lys Phe Pro Phe Thr Phe
            660                 665                 670
Asp Glu Lys Arg Ala Val Gln Gln Leu Arg Ala Cys Gly Val Leu Glu
        675                 680                 685
Thr Ile Arg Ile Ser Ala Ala Gly Phe Pro Ser Arg Trp Thr Tyr Gln
    690                 695                 700
Glu Phe Phe Ser Arg Tyr Arg Val Leu Met Lys Gln Lys Asp Val Leu
705                 710                 715                 720
Ser Asp Arg Lys Gln Thr Cys Lys Asn Val Leu Glu Lys Leu Ile Leu
                725                 730                 735
Asp Lys Asp Lys Tyr Gln Phe Gly Lys Thr Lys Ile Phe Phe Arg Ala
            740                 745                 750
Gly Gln Val Ala Tyr Leu Glu Lys Leu Arg Ala Asp Lys Leu Arg Ala
        755                 760                 765

Ala Cys Ile Arg Ile Gln Lys Thr Ile Arg Gly Trp Leu Leu Arg Lys
    770                 775                 780
Lys Tyr Leu Arg Met Arg Lys Ala Ala Ile Thr Met Gln Arg Tyr Val
785                 790                 795                 800
Arg Gly Tyr Gln Ala Arg Cys Tyr Ala Lys Phe Leu Arg Arg Thr Lys
                805                 810                 815
Ala Ala Thr Ile Ile Gln Lys Tyr Trp Arg Met Tyr Val Val Arg Arg
            820                 825                 830
Arg Tyr Lys Ile Arg Arg Ala Ala Thr Ile Val Leu Gln Ser Tyr Leu
        835                 840                 845
Arg Gly Phe Leu Ala Arg Asn Arg Tyr Arg Lys Ile Leu Arg Glu His
    850                 855                 860
Lys Ala Val Ile Ile Gln Lys Arg Val Arg Gly Trp Leu Ala Arg Thr
865                 870                 875                 880
His Tyr Lys Arg Ser Met His Ala Ile Ile Tyr Leu Gln Cys Cys Phe
                885                 890                 895
Arg Arg Met Met Ala Lys Arg Glu Leu Lys Lys Leu Lys Ile Glu Ala
            900                 905                 910
Arg Ser Val Glu Arg Tyr Lys Lys Leu His Ile Gly Met Glu Asn Lys
        915                 920                 925
Ile Met Gln Leu Gln Arg Lys Val Asp Glu Gln Asn Lys Asp Tyr Lys
    930                 935                 940
Cys Leu Val Glu Lys Leu Thr Asn Leu Glu Gly Ile Tyr Asn Ser Glu
945                 950                 955                 960
Thr Glu Lys Leu Arg Ser Asp Leu Glu Arg Leu Gln Leu Ser Glu Glu
                965                 970                 975
Glu Ala Lys Val Ala Thr Gly Arg Val Leu Ser Leu Gln Glu Glu Ile
            980                 985                 990
Ala Lys Leu Arg Lys Asp Leu Glu Gln Thr Arg Ser Glu Lys Lys Cys

        995                 1000                1005
Ile Glu Glu His Ala Asp Arg Tyr Lys Gln Glu Thr Glu Gln Leu
    1010                1015                1020
Val Ser Asn Leu Lys Glu Glu Asn Thr Leu Leu Lys Gln Glu Lys
    1025                1030                1035
Glu Ala Leu Asn His Arg Ile Val Gln Gln Ala Lys Glu Met Thr
    1040                1045                1050
Glu Thr Met Glu Lys Lys Leu Val Glu Glu Thr Lys Gln Leu Glu
    1055                1060                1065
Leu Asp Leu Asn Asp Glu Arg Leu Arg Tyr Gln Asn Leu Leu Asn
    1070                1075                1080
Glu Phe Ser Arg Leu Glu Glu Arg Tyr Asp Asp Leu Lys Glu Glu
    1085                1090                1095
Met Thr Leu Met Val His Val Pro Lys Pro Gly His Lys Arg Thr
    1100                1105                1110
Asp Ser Thr His Ser Ser Asn Glu Ser Glu Tyr Ile Phe Ser Ser
    1115                1120                1125
Glu Ile Ala Glu Met Glu Asp Ile Pro Ser Arg Thr Glu Glu Pro
    1130                1135                1140
Ser Glu Lys Lys Val Pro Leu Asp Met Ser Leu Phe Leu Lys Leu
    1145                1150                1155
Gln Lys Arg Val Thr Glu Leu Glu Gln Glu Lys Gln Val Met Gln
    1160                1165                1170
Asp Glu Leu Asp Arg Lys Glu Glu Gln Val Leu Arg Ser Lys Ala
    1175                1180                1185
Lys Glu Glu Glu Arg Pro Gln Ile Arg Gly Ala Glu Leu Glu Tyr
    1190                1195                1200
Glu Ser Leu Lys Arg Gln Glu Leu Glu Ser Glu Asn Lys Lys Leu
    1205                1210                1215

Lys Asn Glu Leu Asn Glu Leu Arg Lys Ala Leu Ser Glu Lys Ser
    1220                1225                1230
Ala Pro Glu Val Thr Ala Pro Gly Ala Pro Ala Tyr Arg Val Leu
    1235                1240                1245
Met Glu Gln Leu Thr Ser Val Ser Glu Glu Leu Asp Val Arg Lys
    1250                1255                1260
Glu Glu Val Leu Ile Leu Arg Ser Gln Leu Val Ser Gln Lys Glu
    1265                1270                1275
Ala Ile Gln Pro Lys Asp Asp Lys Asn Thr Met Thr Asp Ser Thr
    1280                1285                1290
Ile Leu Leu Glu Asp Val Gln Lys Met Lys Asp Lys Gly Glu Ile
    1295                1300                1305
Ala Gln Ala Tyr Ile Gly Leu Lys Glu Thr Asn Arg Ser Ser Ala
    1310                1315                1320
Leu Asp Tyr His Glu Leu Asn Glu Asp Gly Glu Leu Trp Leu Val
    1325                1330                1335
Tyr Glu Gly Leu Lys Gln Ala Asn Arg Leu Leu Glu Ser Gln Leu
    1340                1345                1350
Gln Ser Gln Lys Arg Ser His Glu Asn Glu Ala Glu Ala Leu Arg
    1355                1360                1365
Gly Glu Ile Gln Ser Leu Lys Glu Glu Asn Asn Arg Gln Gln Gln
    1370                1375                1380
Leu Leu Ala Gln Asn Leu Gln Leu Pro Pro Glu Ala Arg Ile Glu
    1385                1390                1395
Ala Ser Leu Gln His Glu Ile Thr Arg Leu Thr Asn Glu Asn Leu
    1400                1405                1410
Asp Leu Met Glu Gln Leu Glu Lys Gln Asp Lys Thr Val Arg Lys
    1415                1420                1425
Leu Lys Lys Gln Leu Lys Val Phe Ala Lys Lys Ile Gly Glu Leu

1430                    1435                    1440

Glu Val  Gly Gln Met Glu Asn  Ile Ser Pro Gly Gln  Ile Ile Asp

1445                    1450                    1455

Glu Pro  Ile Arg Pro Val Asn  Ile Pro Arg Lys Glu  Lys Asp Phe

1460                    1465                    1470

Gln Gly  Met Leu Glu Tyr Lys  Lys Glu Asp Glu Gln  Lys Leu Val

1475                    1480                    1485

Lys Asn  Leu Ile Leu Glu Leu  Lys Pro Arg Gly Val  Ala Val Asn

1490                    1495                    1500

Leu Ile  Pro Gly Leu Pro Ala  Tyr Ile Leu Phe Met  Cys Val Arg

1505                    1510                    1515

His Ala  Asp Tyr Leu Asn Asp  Asp Gln Lys Val Arg  Ser Leu Leu

1520                    1525                    1530

Thr Ser  Thr Ile Asn Ser Ile  Lys Lys Val Leu Lys  Lys Arg Gly

1535                    1540                    1545

Asp Asp  Phe Glu Thr Val Ser  Phe Trp Leu Ser Asn  Thr Cys Arg

1550                    1555                    1560

Phe Leu  His Cys Leu Lys Gln  Tyr Ser Gly Glu Glu  Gly Phe Met

1565                    1570                    1575

Lys His  Asn Thr Ser Arg Gln  Asn Glu His Cys Leu  Thr Asn Phe

1580                    1585                    1590

Asp Leu  Ala Glu Tyr Arg Gln  Val Leu Ser Asp Leu  Ala Ile Gln

1595                    1600                    1605

Ile Tyr  Gln Gln Leu Val Arg  Val Leu Glu Asn Ile  Leu Gln Pro

1610                    1615                    1620

Met Ile  Val Ser Gly Met Leu  Glu His Glu Thr Ile  Gln Gly Val

1625                    1630                    1635

Ser Gly  Val Lys Pro Thr Gly  Leu Arg Lys Arg Thr  Ser Ser Ile

1640                    1645                    1650

Ala Asp Glu Gly Thr Tyr Thr Leu Asp Ser Ile Leu Arg Gln Leu
    1655                1660                1665
Asn Ser Phe His Ser Val Met Cys Gln His Gly Met Asp Pro Glu
    1670                1675                1680
Leu Ile Lys Gln Val Val Lys Gln Met Phe Tyr Ile Ile Gly Ala
    1685                1690                1695
Ile Thr Leu Asn Asn Leu Leu Leu Arg Lys Asp Met Cys Ser Trp
    1700                1705                1710
Ser Lys Gly Met Gln Ile Arg Tyr Asn Val Ser Gln Leu Glu Glu
    1715                1720                1725
Trp Leu Arg Asp Lys Asn Leu Met Asn Ser Gly Ala Lys Glu Thr
    1730                1735                1740
Leu Glu Pro Leu Ile Gln Ala Ala Gln Leu Leu Gln Val Lys Lys
    1745                1750                1755
Lys Thr Asp Asp Asp Ala Glu Ala Ile Cys Ser Met Cys Asn Ala
    1760                1765                1770
Leu Thr Thr Ala Gln Ile Val Lys Val Leu Asn Leu Tyr Thr Pro
    1775                1780                1785
Val Asn Glu Phe Glu Glu Arg Val Ser Val Ser Phe Ile Arg Thr
    1790                1795                1800
Ile Gln Met Arg Leu Arg Asp Arg Lys Asp Ser Pro Gln Leu Leu
    1805                1810                1815
Met Asp Ala Lys His Ile Phe Pro Val Thr Phe Pro Phe Asn Pro
    1820                1825                1830
Ser Ser Leu Ala Leu Glu Thr Ile Gln Ile Pro Ala Ser Leu Gly
    1835                1840                1845
Leu Gly Phe Ile Ser Arg Val
1850 1855 .

7. Utilisation d'un kit de réactifs dans la détermination *in vitro* du syndrome des antiphospholipides **caractérisée en ce que** le kit de réactifs comporte la protéine MYOSA, dans laquelle la présence d'anticorps contre la protéine MYOSA dans des échantillons biologiques est détectée, les échantillons biologiques étant sélectionnés parmi du sérum, du plasma ou du liquide tissulaire obtenu à partir d'un sujet.

8. Utilisation d'un kit de réactifs dans la détermination *in vitro* du syndrome des antiphospholipides, selon la revendication 7, **caractérisée en ce que** les anticorps contre la protéine MYOSA comprennent au moins une parmi IgG, IgM ou IgA.

9. Utilisation d'un kit de réactifs dans la détermination *in vitro* du syndrome des antiphospholipides, selon l'une quelconque des revendications 7 et 8, **caractérisée en ce que** les anticorps contre la protéine MYOSA sont détectés par un procédé de détection sélectionné parmi l'un quelconque parmi ELISA, la chimiluminescence ou POCT.
